# EUROPEAN PATENT SPECIFICATION

(11) **EP 1 865 865 B1**
(45) Date of publication and mention of the grant of the patent: **18.01.2012**
(21) Application number: 06733285.8
(22) Date of filing: 10.04.2006
(51) Int. Cl.: A61B 17/72, A61B 17/64

(54) **SURGICAL FIXATION PIN**
CHIRURGISCHER FIXIERSTIFT
BROCHE DE FIXATION CHIRURGICALE

(30) Priority: 08.04.2005 SE 0500774
(43) Date of publication of application: 19.12.2007
(73) Proprietor: Eulogic AB, 582 77 Linköping (SE)
(72) Inventor: Ullman, Michael, 439 33 Onsala (SE); Ekman, Carl, 439 31 Onsala (SE)
(74) Representative: Cederbom, Hans Erik August
(86) International application number: PCT/SE2006/000429
(87) International publication number: WO 2006/107264

(56) References cited:
- EP-A- 0 693 272
- WO-A-03/068080
- FR-A1- 2 728 155
- US-B1- 6 579 293

## Description

The submitted invention relates to a pin of spring material for usage in fractures in bones in arms or other body parts to provide support of the aforementioned bone while healing the fracture, whereby the pin at opposite ends presents a single angled front intramedullary section and has a rear double bent extracortical support extramedullary section provided with an anchor eye, and that the intramedullary section and the extramedullary section are essentially parallel in relation to each other.

It is generally known within healthcare with the treatment of fractured bones that means are used to allow reinforcement and support to the area surrounding a fracture in the bone in question.

### Background

Fractures of the distal forearm (wrist fractures) are the most common of all fractures (annual incidence about 3000 /1 000 000 inhabitants in the industrialised world) and constitutes by it's abundance a major therapeutic problem. Simple fractures are treated with just a plaster while more complex fractures often require open reduction and plate fixation. For a large number of the intermediate complex fractures the choice of treatment is less obvious; while plate fixation may be a too extensive and expensive procedure plaster immobilisation may be insufficient to hold fracture fragments in the desired position while the fracture heals. Other therapeutic modalities also have their draw-backs: classic external fixation immobilises the wrist joint during treatment and wrist stiffness may ensue. To avoid this, the fixation is often removed before the fracture is consolidated which may lead to secondary displacement of the fracture. Another method is to use straight fine wires (0 1.5 - 2.0 mm) drilled into the fragments or introduced through the fracture site. While this is a simple and minimally invasive procedure it requires power tools. Also the wires, being left protruding through the skin, have to be removed before the patient is able to fully mobilise her wrist.
The second bone of the forearm, the ulna, is notoriously difficult to fix and fractures of the distal ulna are therefore often neglected.

Pins have then been used, among others to nail together the bone or have been inserted in the bone's inner canal. The aforementioned pin has then either been allowed to remain in place after the fractured bone has healed or it has been extracted afterwards. Plates similar to angle iron have also been used and which are screwed to the bone by the fracture. Examples of such aids are shown in WO 01/56452 A2.

It has been difficult to operate in these supports by the fracture area and even more difficult to remove these. Neither have these supports constituted an especially good pin in themselves, i.e. they have not interacted with the bone to achieve contact against the same during simultaneous tensioning of the pin. The introduction of the pin in the bone has not been facilitated with similarly known pins and neither has the screwing of these been proven to be easy to achieve.

It is difficult to insert these pins in the bone, and sometime a power drill is required. It is also difficult to securely anchor the pin in the bone, which is why they frequently slide out. In addition, they are frequently left protruding from the skin, with the risk of both inflammation around the pin as well as infection which can spread to the bone and develop into osteomyelitis.

The principal object of the present invention is thus primarily to produce a pin that is suitable for use in the healing of fractured bones in the arms or other parts of the body and which solves, among others, the problems identified above and which is easy and cost effective to manufacture.

The aforementioned purpose is achieved by means of a pin according to the present invention, which is essentially characterised in that the pin's centre section is formed by an intermediate section that presents at its rear end an essentially perpendicularly directed extension in relation to the intramedullary section and to the double bent extracortical support section, and in that said extracortical support extramedullary section formed from said double bent end section of said pin extends from the intermediate section and has two rear pin end sections lying close to each other.

Previously disclosed through FR 2728155 A1 is a so-called intramedullary nail.

Further EP 0693272 A (A2) discloses a pin of spring material for usage in fractures in bones in arms or other body parts to provide support of the aforementioned bone while healing the fracture. The known pin presents at opposite ends in an intramedullary section an angled front section and has in an extramedullary section a double bent extracortical section provided with an anchor eye. The intramedullary section and the extramedullary section being essentially parallel in relation to each other.

Also WO 03/068080 A discloses a similar pin of spring material for usage in fractures in bones to provide support of the aforementioned bone while healing the fracture.

Both disclose pins where the intramedullary pin section and the extramedullary pin section stretch in the same direction.

Known pins don't make it possible to stabilise fractured bone in an easy way both inside and outside the bone and to make it possible to lie flush against the outer cortex of the distal fragment and serving as a support, stabilising the fracture on both side of the same.

### The Invention

The present invention is intended for fixation of moderately complex fractures. The present implants are specifically designed for fixation of wrist fractures, but with modifications it's use can be extended to other fractures. The implant is made of 1.6 mm wire (but other materials or dimensions may be used) with mechanical properties suitable for this particular use. The larger part of the implant is introduced through the fracture line into the intramedullary canal of the main body of the fractured bone and thus becomes stabilised. The lesser part of the implant, anatomically shaped to lie flush against the outer cortex of the distal fragment, stabilises the fracture by serving as a support. Since the implant is introduced through the fracture line and into the intramedullary canal no power tools are needed. The implant has a low profile and is anatomically shaped and does therefore not normally require removal - a second procedure is avoided and the fracture is supported during the consolidation period even while normal activity with the wrist and hand is resumed.

### The implants

### A. Implants for the radius

The implants meant to fix fractures of the radius have the same basic configuration and functions, small variations in the design are made with respect to variations the anatomy of the specific site where they are used.
i. The intramedullary portion is straight but has a curved tip with a rounded end to facilitate its introduction into the intramedullary canal.
ii. The extramedullary portion is shaped to follow the anatomy of the outer cortex of the radius. It is constituted of a double wire connected by a 180 deg distal bend made into the shaped of a hoop. This design enlarges the supporting interface between implant and bone.
iii. The extramedullary and intramedullary parts are roughly parallel but not coaxial - they are connected by an intermediate part at about 90 deg angle to each of the other parts. The length of the intermediate part corresponds to the thickness of the cortical wall of the radius at the fracture site. This transverse part prevents the implant from sliding out of place.
iv. One of the double wires is extended beyond the connecting part to form a fork with the intramedullary part. This is meant to stabilise the implant against the outer wall of the main fracture body.
v. The hoop formed at the distal end of the implant is shaped to fit a screw which can be optionally used to stabilise the distal fragment.

### B Implants for the ulna

The ulnar implant differs from the radial implant in the respect that it is intended to be fixed with one or two screws.
i. The intramedullary portion is straight and has a pointed tip to allow its introduction through the distal part of the ulna into the intramedullary canal
ii. The extramedullary portion has two hoops, the proximal one for screw fixation of the implant itself to the shaft of the ulna, the distal one for optional screw fixation of the distal fragment.
iii. The distal fragment is fixed by being sandwiched between the intramedullary and extramedullary portion of the implant.

The invention is described in the following through a number of preferred embodiments, whereby reference is made to the enclosed drawings, in which
Fig. 1 schematically shows a pin according to the invention implanted in a forearm,
Fig 2 shows different views of a pin according to a first preferred embodiment,
Fig 3 and 4 show different plan views of the aforementioned first pin,
Fig 5 shows different views of a pin according to a second preferred embodiment,
Fig. 6-10 show different plan views of the aforementioned second pin,

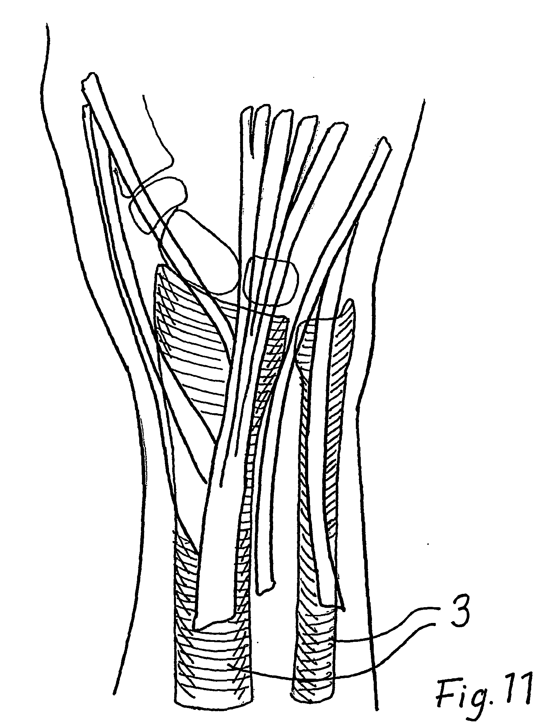

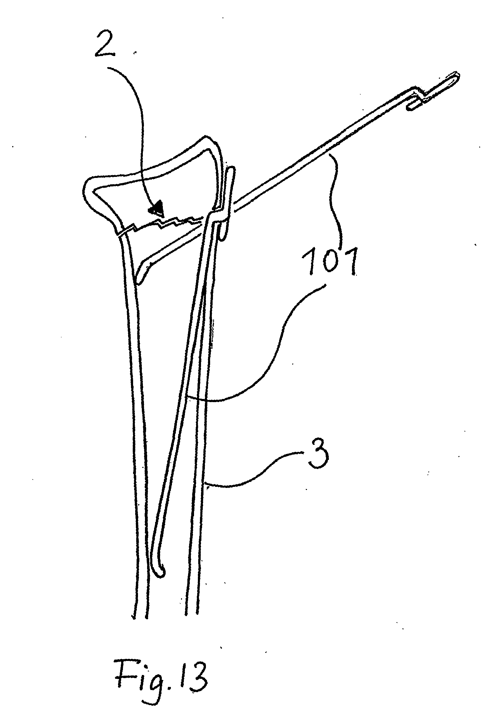

Fig. 11-14 show different examples of the assembly of different types of pin according to the invention,

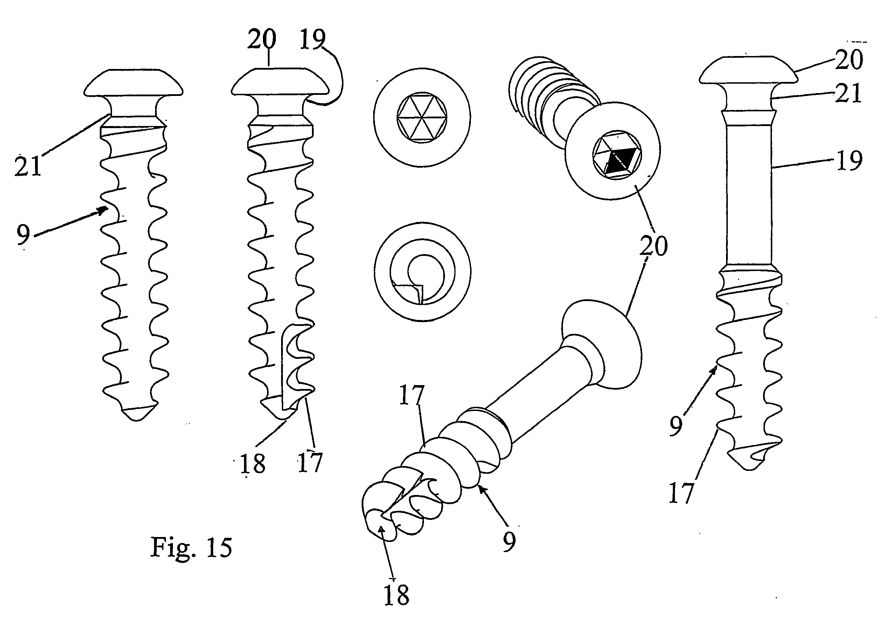

Fig. 15 shows different views of the bone fixation screw according to the invention, and
Fig. 16 schematically shows a sketch of how a pin is applied in a fracture and thereafter inserted in position.

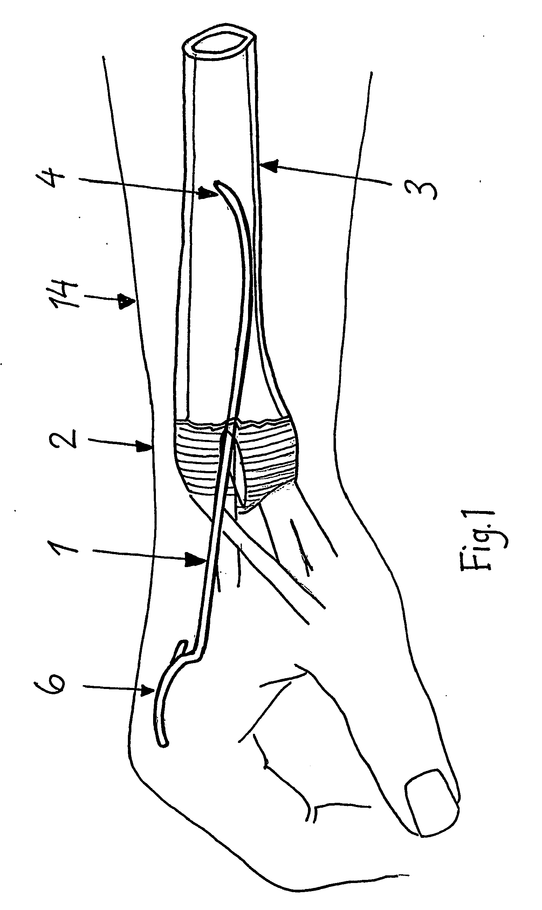

A pin 1 according to the invention is included in a simple and effective system to be used as an aid when treating irregular distal radius and ulna fractures 2. The pin 1 is arranged to achieve maximal stability by using the smallest possible operation and implant. Pin 1 is further arranged to make it easy to insert through the fracture 2 and will be distinctive when inserted against the edge of the fracture in the proximal bone fragment in position in bone 3. The fixing device in the form of bone screw 9 is ideal to be utilized together with an aforementioned pin to further achieve the stability of the fractures. The insertion of the pin 1 is done through a small incision. This is shown in fig. 1.

A pin 1;101;201 that is ideal for use with fracture 2 on bone 3 in arms 14 or other body parts to achieve support and stability to the aforementioned bone 3 when healing the fracture 2 in question is formed according to the present invention principally of three different designs. The aforementioned different pin 1;101;201 are called "Radius Contour Pin", "Radius dorsal Pin" and "Ulna Pin".

In accordance with the invention, the characteristic for all of these pins is that at opposite ends 1A, 1B; 101A,101B and 201A and 201B has an angled rounded front part 4;104 and 204 and a double bend with anchor eye 5;105;205 equipped rear support part 6;106;206, where the pin sections 7A, 7B; 107A, 107B; 207A,207B lie parallel and in close contact with each other. The aforementioned pin 1;101;201 is formed of a spring material, preferably spring steel.

The aforementioned support section 6;106;206 is formed by a, from the centre section of the pin 1C;101C;201C, with blunt angle bent section 8;108;208 and from the aforementioned bent section an extending end section, which is double bent with each pair of pin end sections 7A, 7B; 107A, 107B; 207A, 207B lying tight next to each other.

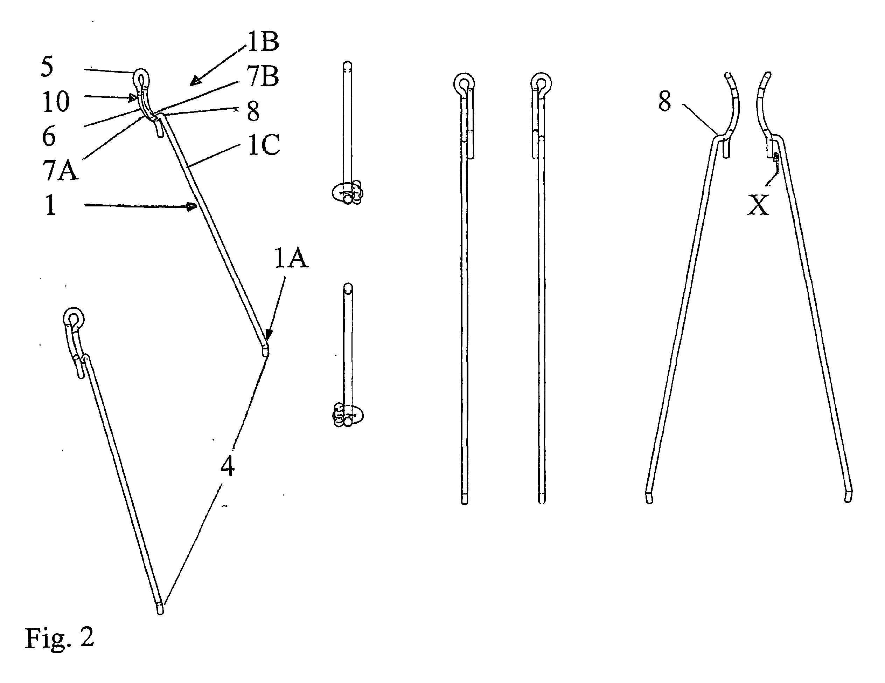

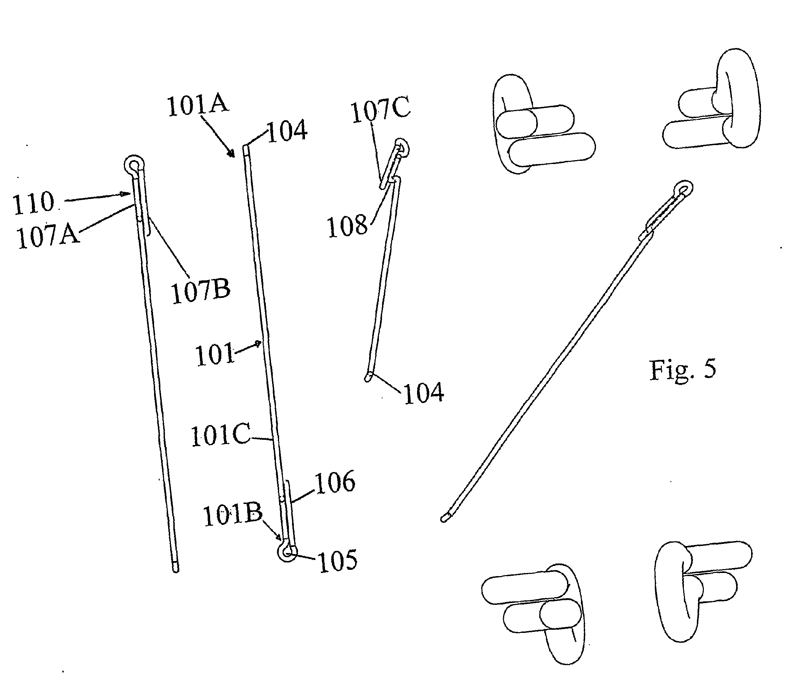

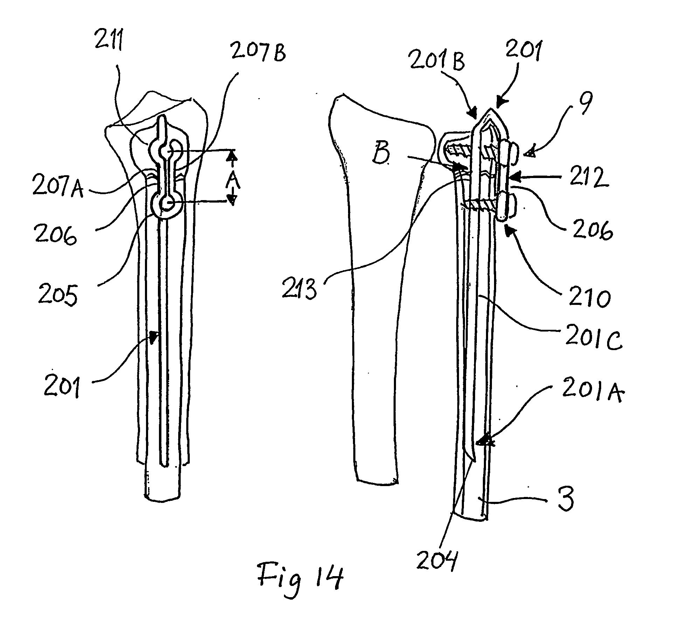

Both pin end parts 7A, 7B; 107A, 107B) ; 207A,207B are straight (fig. 5 and fig. 14) or curved (Fig. 2).

Fig. 14 shows a variant where the pin 201 is bent back at the end 201B principally 180° and is especially suitable for anchorage with screw 9 to the bone 3 in question. A hole shaped outer eye 5;105;205, designed to receive a fixation screw 9 or some other fixing device in that is located by the pin's 1;101;201 and the both pin end sections' outer end and ends 10;110;210 respectively.

In that connection a further inner located eye 211 can be arranged, which is designed to receive a further screw, etc in that, and which is located at a distance A from the first aforementioned outer eye 205.

The section of the pin 206 which comprises the aforementioned screw eyes 205,211 is bent back 180° so that the reformed section 212 is located parallel with the remaining section 213 of the pin 201 and is kept at the distance B from there, as shown in Fig. 14. On the other pin 1;101 the aforementioned blunt angle is essentially right-angled. The function of the aforementioned bending back is for the pin to be kept constantly in position in fracture 2 with the pin's transversal section extending across the bone's 3 linear extension and with the pin's both parallel pin sections arranged to extend internally in and externally on the bone respectively.

The aforementioned angled rounded end section 4;104;204 and the aforementioned bent section 6;106;206 extend along each plane 15,16 which are essentially arranged at right angles in relation to each other.

The pin is principally formed of flat spring steel design, with principally circular cross section, e.g., titanium, stainless steel, plastic, resorbing material or some sort of composite plastic.

Screw 9 which is arranged to secure pin 1;101;201 in position exhibits threads 17 from the tip of the screw 18 up to a plain section 19 which is located next to or at a distance from the screw head 20. A ring shaped receiving section 21 is thereby arranged on the screw 9 to in the secured position be able to be surrounded by the ring shaped eye 5;105;205 and clicked in position therein.

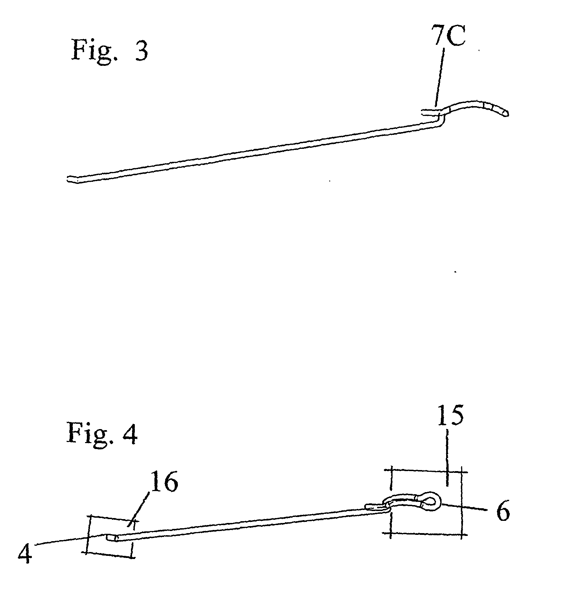

Depending on the type of fracture on the bone 3 that has occurred at the time of the accident, different pins of the type stated above are used and with that the application of the same can vary. The pin according to the examples shown in Fig. 1-4 is made up of a so-called "Radius Contour Pin"1 and this type of pin is inserted I through the actual radial fracture 2. The thereby bent rear support section 6 of the pin 1 will lie tight II against the bone 3 and minimise irritation of nerves and tendons. The extended section, located outside of bone 3, of the rear support section 6 produces good support against the close lying fragment of the bone 3. The possibility of additional fixation with screw 9 etc also exists. The round tip 4 facilitates for the pin to slide against the inside wall of the cortex.

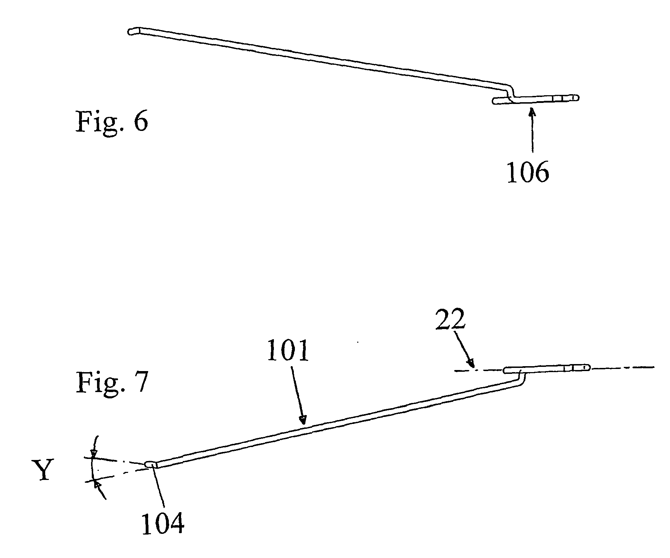

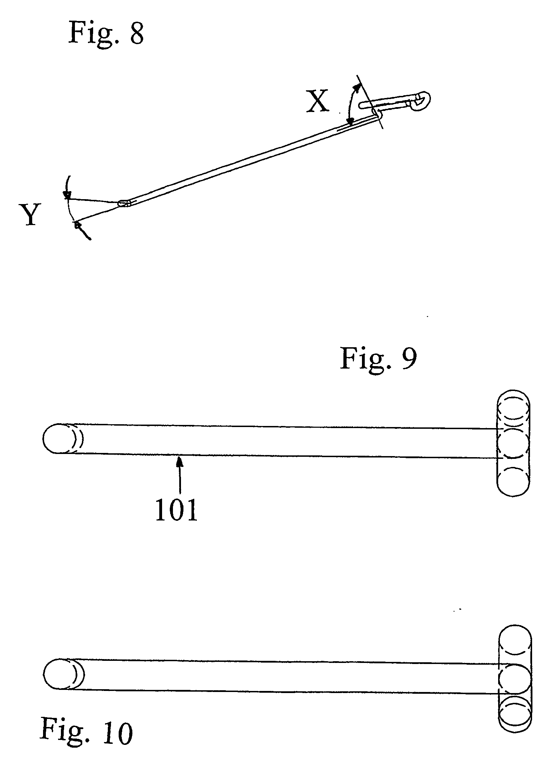

The pin according to the example as shown in Fig. 5-10 makes up a so-called "Dorsal Pin" 101 and such a pin is arranged to be inserted through the "dorsal" fracture line and is arranged to support the fragment with the support section 106 located at the rear of the pin 101. The double rear pin sections 107A,107B on the aforementioned rear support section 106 distribute the pressure across a large area and reduce the risk of cutting into the patient's brittle bone. The possibility of extra bone fixation with screws also exists on this pin 101. The dorsal shank on the pin provides good support to the distal fragment and the transversal bend back prevents the pin from starting to move. The extra cortical shank, i.e. the section of the pin which is arranged to come outside of the bone cortex on the bone, exist in different lengths in order to be varied depending on different types of fracture position. Even this pin is possible to anchor with screws, to further increase stability.

Through the free end 7C;107C of one pin end section 7B;107B of both pin end sections 7A, 7B; 107A,107B extends past the bent section 8;108 of the pin 1;101 effective contact and support is achieved against bone 3 with the aforementioned protruding pin end section.

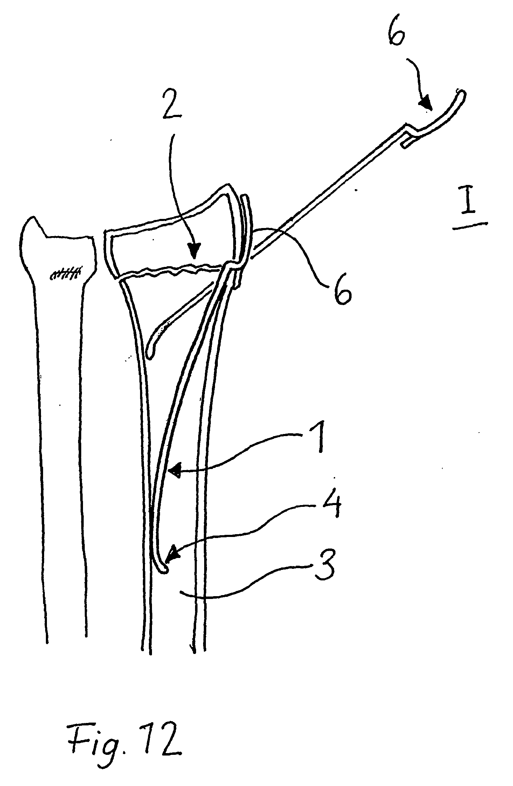

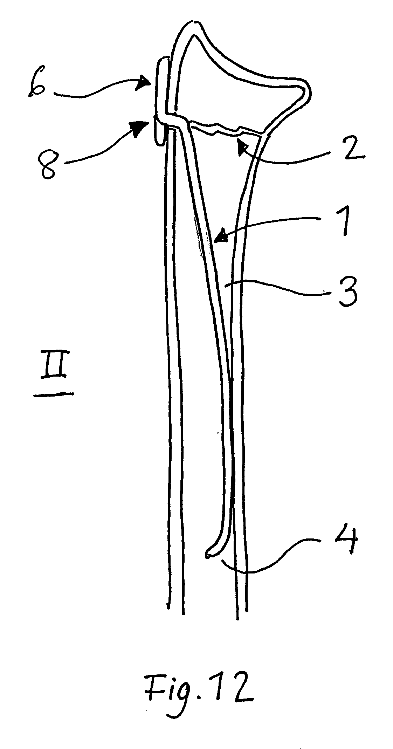

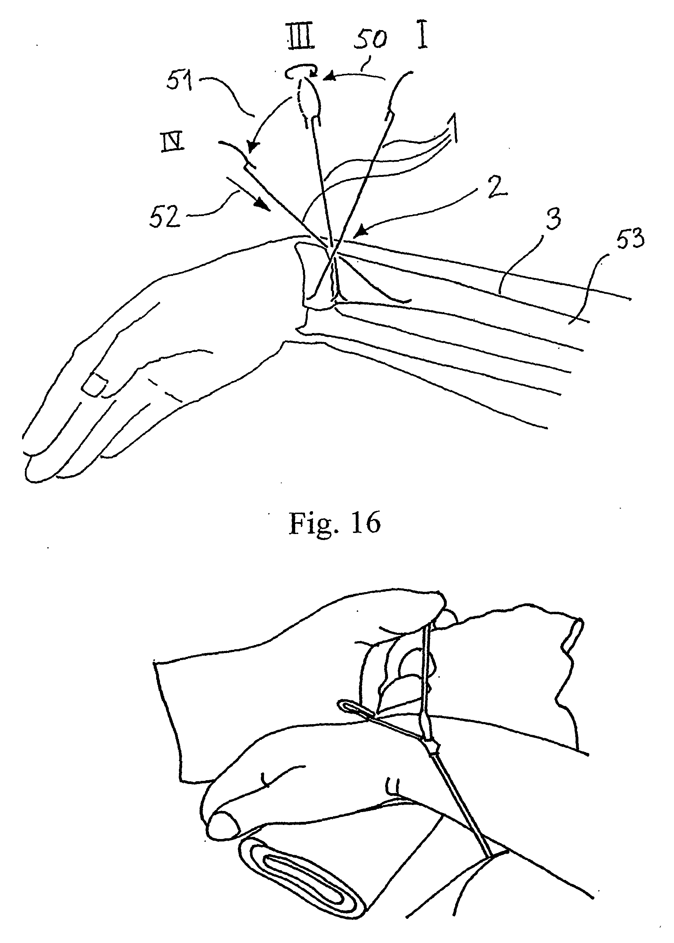

In fig. 16 it is shown how the pin 1 is first inserted through the fracture 2 in a forearm 14 across the bone's 3 longitudinal direction. After that the pin 1 is turned in the direction of arrow 50 to an intermediate position III at which the pin is primarily turned 180° whereupon continued turning of pin 1 occurs in the direction of arrow 51 to the final position IV from which pin 1 is pressed down with its front section in the direction of arrow 52 internally in the bone-marrow 53 in the bone 3 so far that the angled section 8 on the pin 1 extends across through the fracture 2 and continues with its rear section to rest against the outside of the bone 3 as shown in fig. 12 with the final position II. If the fracture is simple, it can be sufficient with one pin but otherwise two or more pins must be used to effectively hold the fracture 2 together.

Finally in Fig. 14 examples of a so-called "Ulna Pin" 201 are shown which is used with extremely complex fractures with many small bone fragments such as a malpositioned fracture sometimes with several fragments on the lower part of the elbow bone. The pin 201 is inserted through the fracture's distal section into the medullary canal of the ulna. It is then secured with screw 9 in the bone and which is received in the screw eyes 205, 211 on the back bent rear section 212 of the pin 201 in the part of the bone that has not been fractured. The pin is suitable to be left in the bone in the form of an implant.

On account of the pin's spring properties, the pins stabilises the fragment of the fracture through the tension between the distal cortex and the proximal medullary canal. The pin is inserted until the traverse section of the pin snaps into the fracture's fracture line.

Additional advantages offered by the invention is that the pin is anatomically designed so that it lies tight against the bone and by that effectively increases the contact surface against the contact section of the bone.

Consequently, the pin is ideal to be utilized both to position the fracture into he right position and to hold together the bone fragments.

## Claims

1. Pin (1;101;201) of spring material for usage in fractures (2) in bones (3) in arms (14) or other body parts to provide support of the aforementioned bone (3) while healing the fracture, whereby the pin (1;101;201) at opposite ends (1A,1B;101A,101B;201A,201B) presents respectively a single angled front intramedullary section (4;104;204) and a rear double bent extracortical support extramedullary section (6;106;206) provided with an anchor eye (5;105;205), the intramedullary section and the extramedullary section being essentially parallel in relation to each other, **characterised in that**, the pin's centre section (1C;101C;201C) is formed by an intermediate section (8;108;208) that presents at its rear end an essentially perpendicularly directed, extension in relation to the intramedullary section and to the double bent extracortical support extramedullary section (6; 106; 206), and **in that** said extracortical support extramedullary section (6;106;206) formed from said double bent end section of said pin extends from the intermediate section (8; 108; 208) and has two rear pin end sections (7A, 7B; 107A, 107B; 207A, 207B) lying close to each other.

2. Pin according to claim 1, **characterised in that**, both pin end sections (107A,107B;207A,207B) are straight.

3. Pin according to claim 1, **characterised in that**, both pin end sections (7A,7B) are curved.

4. Pin according to any of claims 1-3, **characterised in that**, the anchor eye (5;105;205), intended to receive a fixation screw (9) or other fixation device therein, is located by the both pin end section's outer ends (10;110;210).

5. Pin according to claim 4, **characterised in that**, an additional inner eye (211), intended to receive a screw (9) etc, therein, is located at a distance (A) from the first aforementioned outer eye (205).

6. Pin according to any of the claims 1-5, **characterised in that**, the aforementioned front section (4;104;204) and the aforementioned intermediate section extend along separate planes that are essentially perpendicularly arranged in relation to each other.

7. Pin according to any of claims 1-6, **characterised in that**, the pin is formed of a flat spring steel design.

8. Pin according to any of the claims 1-4 or 7-8, **characterised in that**, the free end (7C;107C) of one (7B;107B) of the two pin end sections (7A,7B;107A,107B) extends past the intermediate section (8;108) of the pin.

9. Pin according to any of the claims 4-8, further comprising a screw and which is arranged to be fixed with the help of said screw, **characterised in that**, the screw (9) presents threads (17) up to a plain section (19) next to or at a distance from the screw head (20) where a ring shaped reception section (21) is arranged to be enclosed by the ring shaped eye (5;105;205).

10. Pin according to any of the claims 4-8, **characterised in that**, the screw presents threads all the way up to the screw head and that the aforementioned threads are designed to work together with the pin's round cross section part.

11. Pin according to any of the claims 1-10, **characterised in that**, that the front section of said pin is angled (4) between 10°-30° with respect to the center portion and that it is angled with respect to the rear double bent support section's common plane (22).

## Patentansprüche

1. Stift (1; 101; 201) aus Federmaterial zum Gebrauch bei Brüchen (2) in Knochen (3) in Armen (14) oder anderen Körperteilen, um eine Abstützung des Knochens (3) bereitzustellen, während der Bruch heilt, wobei der Stift (1; 101; 201) an gegenüberliegenden Enden (1A, 1B; 101A, 101B; 201A, 201B) einen einzelnen angewinkelten vorderen intramedullären Abschnitt (4; 104; 204) bzw. einen rückwärtigen doppelten gebogenen extrakortikalen extramedullären Stützabschnitt (6; 106; 206) aufweist, der mit einer Verankerungsöse (5; 105; 205) versehen ist, wobei der intramedulläre Abschnitt und der extramedulläre Abschnitt im Wesentlichen parallel in Bezug zueinander angeordnet sind,
**dadurch gekennzeichnet , dass**
der Zentralabschnitt des Stiftes (1C; 101C; 20 1 C) durch einen Zwischenabschnitt (8; 108; 208) geformt ist, der an seinem rückwärtigen Ende eine im Wesentlichen rechtwinklig gerichtete Verlängerung in Bezug auf den intramedullären Abschnitt und den doppelt gebogenen extrakortikalen extramedullären Stützabschnitt (6; 106; 206) aufweist, und dass sich der extrakortikale extramedulläre Stützabschnitt (6; 106; 206), der aus dem doppelt gebogenen Endabschnitt des Stiftes geformt ist, von dem Zwischenabschnitt (8; 108; 208) erstreckt und zwei rückwärtige Stiftendabschnitte (7A, 7B; 107A, 107B; 207A, 207B) aufweist, die eng aneinander liegen.

2. Stift nach Anspruch 1,
**dadurch gekennzeichnet , dass**
beide Stiftendabschnitte (107A, 107B; 207A, 207B) gerade sind.

3. Stift nach Anspruch 1,
**dadurch gekennzeichnet , dass**
beide Stiftendabschnitte (7A, 7B) gekrümmt sind.

4. Stift nach einem der Ansprüche 1-3,
**dadurch gekennzeichnet , dass**
die Verankerungsöse (5; 105; 205), die dazu bestimmt ist, eine Fixierungsschraube (9) oder eine andere Fixierungsvorrichtung darin aufzunehmen, durch die beiden Außenenden des Stiftendabschnittes (10; 110; 210) angeordnet ist.

5. Stift nach Anspruch 4,
**dadurch gekennzeichnet, dass**
eine zusätzliche Innenöse (211), die dazu bestimmt ist, eine Schraube (9) etc. darin aufzunehmen, unter einer Distanz (A) von der ersten Außenöse (205) angeordnet ist.

6. Stift nach einem der Ansprüche 1 - 5,
**dadurch gekennzeichnet , dass**
der Vorderabschnitt (4; 104; 204) und der Zwischenabschnitt sich entlang separater Ebenen erstrecken, die im Wesentlichen rechtwinklig in Bezug zueinander angeordnet sind.

7. Stift nach einem der Ansprüche 1 - 6,
**dadurch gekennzeichnet, dass**
der Stift aus einer Konstruktion aus flachem Federstahl geformt ist.

8. Stift nach einem der Ansprüche 1 - 4 oder 7 - 8,
**dadurch gekennzeichnet , dass**
das freie Ende (7C; 107C) von einem (7B; 107B) der beiden Stiftendabschnitte (7A, 7B; 107A, 107B) sich an dem Zwischenabschnitt (8, 108) des Stiftes vorbei erstreckt.

9. Stift nach einem der Ansprüche 4-8,
ferner mit einer Schraube und der derart angeordnet ist, dass er mit Hilfe der Schraube befestigt werden kann,
**dadurch gekennzeichnet, dass**
die Schraube (9) ein Gewinde (17) bis zu einem ebenen Abschnitt (19) benachbart oder unter einer Distanz von dem Schraubenkopf (20) aufweist, wobei ein ringförmiger Aufnahmeabschnitt (21) so angeordnet ist, dass er durch die ringförmige Öse (5; 105; 205) umschlossen ist.

10. Stift nach einem der Ansprüche 4-8,
**dadurch gekennzeichnet, dass**
die Schraube ein Gewinde über den gesamten Weg bis zum Schraubenkopf aufweist und dass das Gewinde derart ausgelegt ist, dass es mit dem Teil mit rundem Querschnitt des Stiftes zusammenwirkt.

11. Stift nach einem der Ansprüche 1-10,
**dadurch gekennzeichnet, dass**
der Vorderabschnitt des Stiftes zwischen 10° - 30° in Bezug auf den Zentralabschnitt angewinkelt (4) ist und dass er in Bezug auf die gemeinsame Ebene (22) des rückwärtigen doppelten gebogenen Stützabschnittes angewinkelt ist.

## Revendications

1. Broche (1 ; 101 ; 201) pour matériau élastique ressort destiné à être utilisée pour des fractures (2) d'os (3) des bras (14) ou d'autres parties du corps afin de fournir un support pour l'os (3) susmentionné pendant la guérison de la fracture, laquelle broche (1 ; 101 ; 201) présente respectivement au niveau de ses extrémités opposées (1A, 1 B ; 101A, 101 B ; 201A, 201 B) une partie intramédullaire avant (4 ; 104 ; 204) avec un seul coude et une partie extramédullaire arrière (6 ; 106 ; 206) de support à double courbure pourvue d'un oeillet d'ancrage (5 ; 105 ; 205), la partie intramédullaire et la partie extramédullaire étant sensiblement parallèles l'une à l'autre, **caractérisée en ce que** la partie centrale de la broche (1 C ; 101 C ; 201 C) est formée par une zone intermédiaire (8 ; 108 ; 208) qui présente au niveau de son extrémité arrière une prolongation sensiblement perpendiculaire à la partie intramédullaire et à la partie extramédullaire arrière (6 ; 106 ; 206) de support à double courbure, et **en ce que** cette partie extramédullaire arrière (6 ; 106 ; 206) de support extracortical formée à partir de la partie d'extrémité à double courbure de ladite broche s'étend à partir de la zone intermédiaire (8 ; 108 ; 208) et présente deux parties d'extrémité de broche arrière (7A, 7B ; 107A, 107B ; 207A, 207B) proches l'une de l'autre.

2. Broche selon la revendication 1, **caractérisée en ce que** les deux parties d'extrémité (107A, 107b ; 207A, 207B) de la broche sont droites.

3. Broche selon la revendication 1, **caractérisée en ce que** les deux parties d'extrémité (7A, 7B) de la broche sont incurvées.

4. Broche selon l'une quelconque des revendications 1 à 3, **caractérisée en ce que** l'oeillet d'ancrage (5 ; 105 ; 205), prévu pour recevoir une vis de fixation (9) ou tout autre dispositif de fixation, est formé par les deux extrémités externes (10 ; 110 ; 210) des parties d'extrémité de la broche.

5. Broche selon la revendication 4, **caractérisée en ce qu'**un oeillet interne supplémentaire (211), prévu pour recevoir une vis (9) etc., est situé à une distance (A) du premier oeillet externe susmentionné (205).

6. Broche selon l'une quelconque des revendications 1 à 5, **caractérisée en ce que** la partie avant (4 ; 104 ; 204) susmentionnée et la zone intermédiaire susmentionnée s'étendent le long de plans distincts sensiblement perpendiculaires.

7. Broche selon n'importe laquelle de revendications 1 à 6, **caractérisée en ce que** la broche est réalisée sous la forme d'un ressort plat en acier.

8. Broche selon l'une quelconque des revendications 1 à 4 ou 7 à 8, **caractérisée en ce que** l'extrémité libre (7C;107C) d'une (7B ; 107B) ou des deux parties d'extrémité de la broche (7A, 7b ; 107a, 107b) se prolonge au-delà de la zone intermédiaire (8 ; 108) de la broche.

9. Broche selon l'une quelconque des revendications 4 à 8, comportant en outre une vis et prévue pour être fixée au moyen de ladite vis, **caractérisée en ce que** la vis (9) présente des filetages (17) jusqu'à une partie plate (19) à proximité immédiate ou à distance de la tête de vis (20) où une partie de réception conformée en anneau (21) est prévue pour être entourée par l'oeillet conformé en anneau (5 ; 105 ; 205).

10. Broche selon l'une quelconque des revendications 4 à 8, **caractérisée en ce que** la vis présente des filetages tout du long et jusqu'à la tête de vis et **en ce que** les filetages susmentionnés sont conformés pour collaborer avec la partie à section ronde de la broche.

11. Broche selon l'une quelconque des revendications 1 à 10, **caractérisée en ce que** la partie avant de ladite broche est déviée d'un angle (4) compris entre 10° et 30° par rapport à la partie centrale et **en ce qu'**elle est en oblique par rapport au plan commun (22) de la partie de support arrière à double courbure (22).
